# EUROPEAN PATENT SPECIFICATION

(11) **EP 4 225 304 B1**
(45) Date of publication and mention of the grant of the patent: **17.12.2025**
(21) Application number: 21880871.5
(22) Date of filing: 12.10.2021
(51) Int. Cl.: C07D 471/10

(54) **SYNTHESIS OF EGFR MODULATORS**
SYNTHESE VON EGFR-MODULATOREN
SYNTHÈSE DE MODULATEURS DE L'EGFR

(30) Priority: 12.10.2020 US 202063090336 P; 12.10.2020 WO PCT/CN2020/120339
(43) Date of publication of application: 16.08.2023
(73) Proprietor: The Regents of The University of Michigan, Ann Arbor, MI 48109-2590 (US)
(72) Inventor: RECH, Jason, Christopher, Ann Arbor, MI 48103 (US); NYATI, Mukesh, K., Superior Township, MI 48198 (US); FANG, Fan, Suzhou Jiangsu, 215006 (CN); ZHANG, Lei, Suzhou Jiangsu, 215006 (CN); HENSCHKE, Julian, Paul, Uraidla, SA 5142 (AU)
(74) Representative: Zacco Sweden AB
(86) International application number: PCT/US2021/054485
(87) International publication number: WO 2022/081514

(56) References cited:
- WO-A1-2019/165358
- US-A1- 2016 016 956
- US-B2- 8 039 460

## Description

### BACKGROUND

The present disclosure relates to processes for synthesizing 2-((3-(4-bromophenyl)-8-methyl-1,4,8-triazaspiro[4.5]deca-1,3-dien-2-yl)thio)-N-(quinolin-3-yl)acetamide (Compound A) and salts thereof:

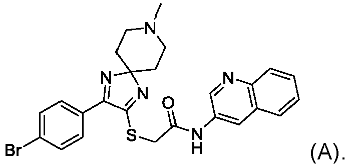

Compound A is a modulator of EGFR useful in treating or preventing diseases or disorders associated with aberrant EGFR activity, e.g., cancer.

The EGFR small molecule tyrosine kinase inhibitors (TKI's) erlotinib, gefitinib, and afatinib have been most successful as single agents in the treatment of lung adenocarcinomas that have somatic mutations (such as L858R or deletion in exon 19, i.e. E746-A750) that confer sensitivity to this class of drugs, which occur in 7-20% of patients depending on ethnicity and gender. Unfortunately, responses rarely last more than a year because virtually all patients develop resistance to therapy. A third-generation irreversible inhibitor, osimertinib (AZD9291), is effective in treating naive as well as patients who have acquired resistance to first or second generation TKls. However, within a year of treatment with osimertinib, a majority of patients develop another mutation in the EGFR kinase domain (C797S), which is the drug covalent attachment site. Although several approaches to target osimertinib resistant EGFR have been reported, as of now no TKI treatment option exists for these patients with this C797S mutation. Chemotherapy is the only option.

In view of the foregoing, there exists a need for a cancer therapeutic that targets EGFR in a manner other than inhibition of EGFR tyrosine kinase activity. There also exists a need for a therapeutic that treats cancer without drug resistance developing after initial use.

PCT Publication No. WO WO2019/165358 discloses compound A as an EGFR modulator and provides a process for preparing it. However, improved synthetic processes that result in greater yield and purity of Compound A are desired, particularly for the commercial production of compound A.

### SUMMARY

Provided herein are processes for synthesizing Compound A, or a salt or solvate thereof: 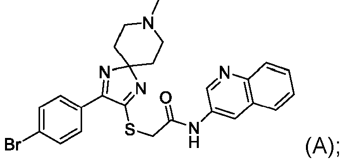 comprising (i) admixing Compound I and a halogenation reagent or a sulfonylation reagent to form Compound II: 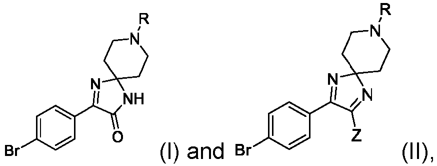 wherein R is methyl or a nitrogen-protecting group and Z is a halogen or a sulfonate group. In some cases, R is methyl. In some cases, R is a nitrogen-protecting group. In some cases, R is t-butyloxycarbonyl (Boc), benzyloxycarbonyl (Cbz), benzyl, or p-methoxybenzyl (PMB). In some cases, Z is a sulfonate group. In some cases, the sulfonate group is triflate, mesylate, tosylate, benzenesulfonate, or nosylate. In some cases, the sulfonate group is triflate.

In some cases, Compound II is synthesized by admixing Compound I and a halogenation reagent. In some cases, the halogenation reagent is a chlorination reagent. In some cases, the chlorination reagent comprises oxalyl chloride, SOCl₂ or POCl₃.

In some cases, Compound II is synthesized by admixing Compound I and a sulfonylation reagent. In some cases, the sulfonylation reagent is selected from triflic anhydride, mesyl chloride, mesic anhydride, tosyl chloride, tosic anhydride, nosyl chloride, and a perfluoroalkylsulfonic anhydride. In some cases, the sulfonylation reagent is triflic anhydride.

In some cases, Compound II is synthesized by admixing Compound I and a halogenation or a sulfonylation reagent in an organic solvent. In some cases, Compound II is synthesized by admixing Compound I and a halogenation or a sulfonylation reagent in a mixture of organic solvents. In some cases, Compound II is synthesized by admixing Compound I and a halogenation or a sulfonylation reagent in a mixture of dichloromethane and ethyl acetate.

In various embodiments, the processes further comprise synthesizing Compound A by (ii) (a) admixing Compound II and Compound III in the presence of a base to form Compound A: 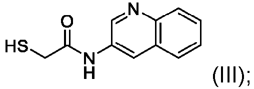 or
(b) (I) admixing Compound II and a nucleophilic sulfuration reagent to form Compound IIA, then (II) admixing Compound IIA with Compound IIIA in the presence of a base to form Compound A: 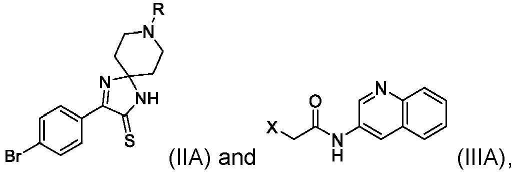 wherein X is halogen.

In some cases, X is Br or Cl. In some cases, X is Cl.

In some cases, the processes comprise synthesizing Compound A by admixing Compound II and Compound III in the presence of a base to form Compound A. In some cases, the base is NaH, NaOH, KOH, sodium methoxide, sodium ethoxide, sodium tert-butoxide, or potassium tert-butoxide.

In some cases, the processes comprise synthesizing Compound A by (b) (I) admixing Compound II and a nucleophilic sulfuration reagent to form Compound IIA, then (II) admixing Compound IIA with Compound IIIA in the presence of a base to form Compound A. In some cases, the nucleophilic sulfuration reagent comprises Li₂S, Na₂S, K₂S, Li₂Sₓ, Na₂Sₓ, K₂Sₓ, (wherein x = 2-5), NaBH₄/S, NaSH, AcSK, thiourea or a salt or hydrate thereof. In some cases, the nucleophilic sulfuration reagent comprises a hydrate of Na₂S. In some cases, the nucleophilic sulfuration reagent comprises Na₂S•4H₂O.

In some cases, the processes comprise synthesizing Compound A by (b)(I) admixing Compound II and a nucleophilic sulfuration reagent in an organic solvent to form Compound IIA. In some cases, the processes comprise synthesizing Compound A by (b)(I) admixing Compound II and a nucleophilic sulfuration reagent in a mixture of organic solvents to form Compound IIA. In some cases the processes comprise synthesizing Compound A by (b)(I) admixing Compound II and a nucleophilic sulfuration reagent in a mixture of dichloromethane and ethyl acetate to form Compound IIA.

In some cases, the processes comprise synthesizing Compound A by (II) admixing Compound IIA with Compound IIIA in the presence of a base to form Compound A. In some cases, the base is K₂CO₃.

In some cases, the processes comprise synthesizing Compound A by (II) admixing Compound IIA with Compound IIIA in the presence of a base in an organic solvent to form Compound A. In some cases, the organic solvent is isopropanol.

In various embodiments, the processes further comprise synthesizing compound A by (iii) optionally, when R is a nitrogen-protecting group, removing the nitrogen-protecting group and methylating the resulting deprotected amine to form Compound A wherein R is methyl. In some cases, when the processes comprise step (iii), R is a nitrogen-protecting group. In some cases, step (iii) is performed between steps (ii)(b)(I) and (ii)(b)(II). In some cases, when R is a nitrogen-protecting group, the process further comprises removing the nitrogen-protecting group from Compound IIA and methylating the resulting deprotected amine to form Compound IIA wherein R is methyl. In some cases, removing the nitrogen-protecting group comprises admixing in the presence of acid. In some cases, the deprotected amine is methylated by admixing with NaBH(OAc)₃, CH₂O, and acetic acid.

Further aspects and advantages will be apparent to those of ordinary skill in the art from a review of the following detailed description. The description hereafter includes specific embodiments with the understanding that the disclosure is illustrative, and is not intended to limit the invention to the specific embodiments described herein.

### DETAILED DESCRIPTION

Provided herein are processes for synthesizing EGFR modulators and salts thereof. In particular, processes for synthesizing 2-((3-(4-bromophenyl)-8-methyl-1,4,8-triazaspiro[4.5]deca-1,3-dien-2-yl)thio)-N-(quinolin-3-yl)acetamide (Compound A) and salts thereof are provided:

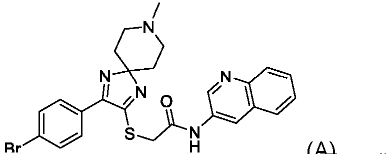

PCT Publication No. WO 2019/165358 (the "'358 (A) application") discloses Compound A as an EGFR modulator and provides a process for preparing it.

The '358 application generally describes a procedure for making compounds such as Compound A as shown in Scheme 1, below, which is adapted from the disclosure at paragraph [0090] (General Procedure A) of the '358 application. The '358 application describes that substituted acetamides 7A-G were added to 3-(4-bromophenyl)-8-methyl-1,4,8-triazaspiro[4.5]dec-3-ene-2-thione (labeled as Compound 6) in anhydrous acetonitrile and warmed to 40 °C. Next, 2M aqueous potassium carbonate solution was added to the reaction mixture and the reaction was maintained at 40 °C until TLC analysis indicated loss of starting materials and a new R_{f} spot (typically 2-6 hours). By following the procedure in Scheme 1 using 2-chloroacetamides 7, substituted 3-(4-bromophenyl)-8-methyl-1,4,8-triazaspiro[4.5]deca-1,3-diene-2-thiols 8A-G were produced, including using 7C (2-chloro-N-quinolin-3-yl-acetamide) to produce Compound A (referred to as "8C" in the '358 application). 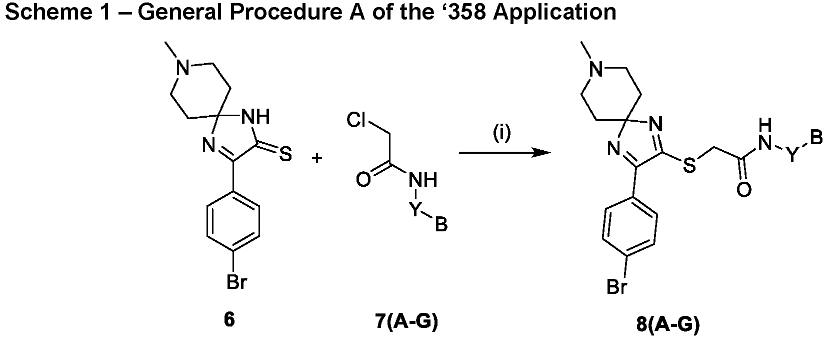
Reaction conditions: (i) 2M aq. K₂CO₃, Acetonitrile, 40°C
Y: C₀₋₆alkylene
B: optionally substituted C₆₋₁₀ aryl, 5-10 membered heteroaryl,
3-8 membered cycloalkyl, or 3-12 membered heterocycloalkyl

The '358 application further describes a process for synthesizing intermediate compounds like compound 6 in Scheme 1. Scheme 2, below, which is adapted from paragraph [0105] (Examples 15-28) of the '358 application, represents the general process of synthesizing substituted 2-(4-bromophenyl)-3-thioxo-1,4,8-triazaspiro[4.5]dec-1-ene compounds (e.g., compound 6) as described in the '358 application. Briefly, to a solution of tert-butyl 2-(4-bromophenyl)-3-oxo-1,4,8-triazaspiro[4.5]dec-1-ene-8-carboxylate was added Lawesson's reagent in THF and the reaction mixture was heated to 60 °C until the reaction was complete by TLC. The reaction mixture was concentrated onto silica gel and purified by flash column chromatography (0-100% EtOAc in hexanes) to provide tert-butyl 2-(4-bromophenyl)-3-thioxo-1,4,8-triazaspiro[4.5]dec-1-ene-8-carboxylate.

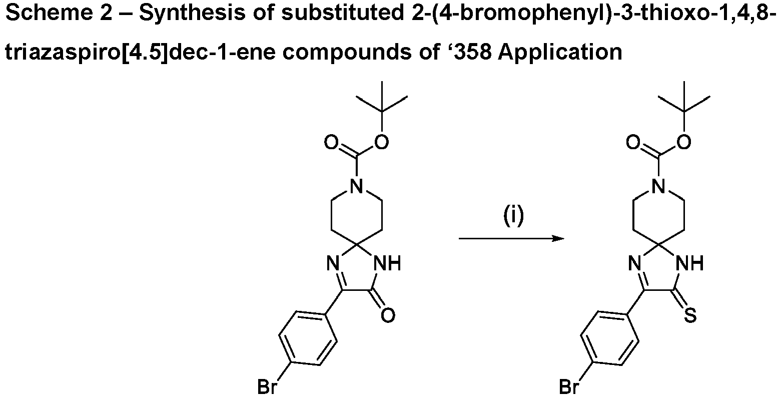

### Reaction conditions: (i) Lawesson's Reagent, THF

The process of the '358 application has several disadvantages for large-scale synthesis. Significantly, the Lawesson's reagent used to convert the amide moiety to a thioamide is malodorous and highly moisture-sensitive, and there are potential difficulties in performing the amide to thioamide conversion at the scale of several hundred grams. The process of the '358 application also employs several column chromatography purification steps, which are impractical for a synthesis at the scale of several hundred grams or larger.

Advantageously, the processes described herein avoid the use of Lawesson's reagent for the preparation of thioamides, and are more practical and higher-yielding. The processes described herein also avoid the necessity of purification by column chromatography.

Described herein are processes for synthesizing Compound A and salts thereof: 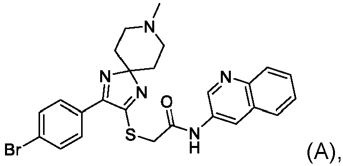 comprising (i) admixing Compound I and a halogenation reagent or a sulfonylation reagent to form Compound II: 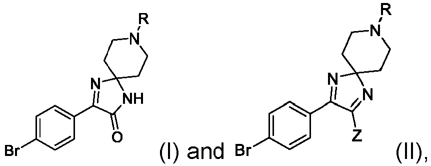 wherein R is methyl or a nitrogen-protecting group and Z is chloride or a sulfonate group;
(ii)
   (a) admixing Compound II and Compound III in the presence of a base to form Compound A: 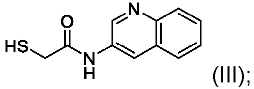 or
   (b) (I) admixing Compound II and a nucleophilic sulfuration reagent to form Compound IIA, then (II) admixing Compound IIA with Compound IIIA in the presence of a base to form Compound A: 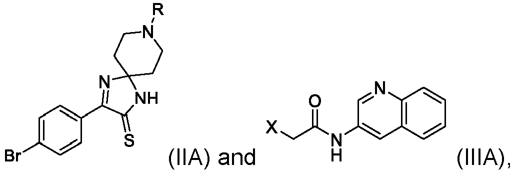 wherein X is Cl or Br, and
(iii) optionally, when R is a nitrogen-protecting group, removing the nitrogen-protecting group and methylating the resulting deprotected amine to form Compound A wherein R is methyl, as discussed in detail below.

As will be appreciated, the disclosed processes involve formation of 3-(4-bromophenyl)-8-methyl-1,4,8-triazaspiro[4.5]deca-1,3-diene-2-thioacetamides by the addition of a thiol group to a 3-halo (e.g., 3-chloro)- or 3-sulfonyloxy-1,4,8-triazaspiro[4.5]deca-1,3-diene, or the addition of a chloro- or bromoacetamide to a 1,4,8-triazaspiro[4.5]dec-3-ene-2-thione.

A general reaction scheme for the processes described herein is provided in Scheme 3, below:

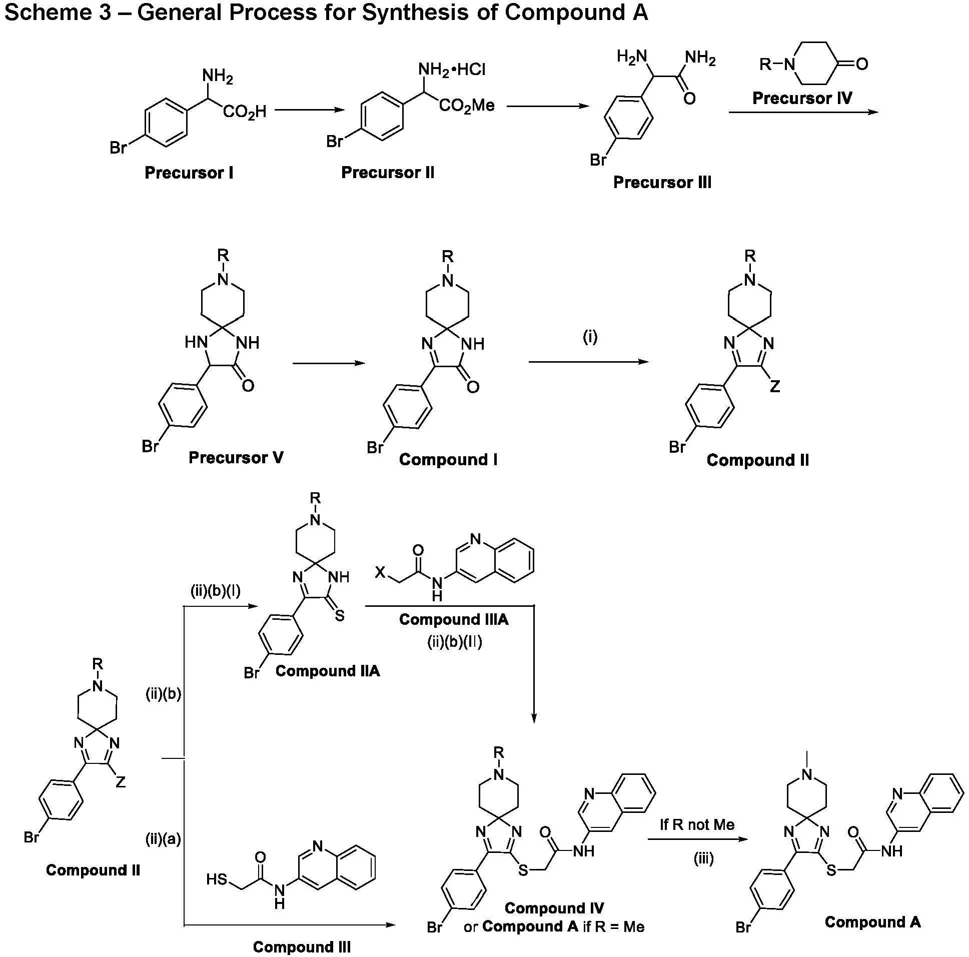

Scheme 3 is not intended to be limiting in scope. For example, when necessary, step (iii) to remove a nitrogen-protecting group for R and replace it with a methyl group for R may precede or follow step (ii)(a) or (ii)(b). In some cases, step (iii) can take place between steps (ii)(b)(I) and (ii)(b)(II).

### Synthesis of Compound II

The processes of the disclosure can include synthesizing Precursor V:

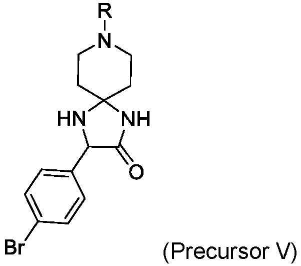

As used throughout, R is methyl or a nitrogen-protecting group. In some cases, R is a CD₃ group (i.e., a deuterated methyl group). Nitrogen-protecting groups are generally known in the art. Nonlimiting examples of nitrogen-protecting groups include carbobenzyloxy (Cbz) groups, acetyl groups, t-butyloxycarbonyl (Boc) groups, and 9-fluorenylmethyloxycarbonyl (Fmoc) groups.

In particular, an α-aminoamide (Precursor III) can be reacted with a piperidin-4-one (Precursor IV) to form a substituted 1,4,8-triazaspiro[4.5]decan-2-one (Precursor V):

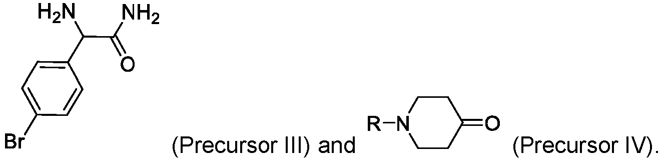

In some embodiments, the formation of Precursor V is carried out in an organic solvent. Organic solvents are generally known in the art. Nonlimiting examples of organic solvents that can be used throughout the processes described herein include ethyl acetate, acetonitrile, toluene, benzene, xylene, chlorobenzene, fluorobenzene, naphthalene, benzotrifluoride, tetrahydrofuran (THF), tetrahydropyran, dimethylformamide (DMF), tetrahydrofurfuryl alcohol, diethyl ether, dibutyl ether, diisopropyl ether, methyl tert-butyl ether (MTBE), 2-methyltetrahydrofuran (2-MeTHF), dimethyl sulfoxide (DMSO), 1,2-dimethoxyethane (1,2-DME), 1,2-dichloroethane (1,2-DCE), 1,4-dioxane, cyclopentylmethyl ether (CPME), chloroform, carbon tetrachloride, dichloromethane (DCM), 2-butanone, methanol, ethanol, propanol, and 2-propanol, or a combination thereof. In some embodiments, the formation of Precursor V is carried out in ethanol. In some embodiments, the formation of Precursor V is carried out at elevated temperature. In some embodiments, the formation of Precursor V is carried out at a temperature of 20 °C to 100 °C, for example, at least 20, 30, 40, 50, 60, 70, 80, 90, or 100 °C and/or up to 60, 70, 80, 90, or 100 °C, such as 20 °C to 80 °C, 40 °C to 80 °C, 50°C to 90°C, 60 °C to 80 °C, 75°C to 85°C, or 70 °C to 80 °C. In some embodiments, the formation of Precursor V occurs at a temperature of 80 °C.

The processes of the disclosure can include synthesizing Compound I.

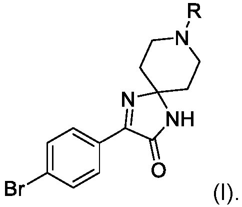

In particular, Precursor V can be oxidized to form Compound I. In some embodiments, Compound I is formed via an addition-elimination reaction. In some embodiments, Compound I is formed by treating Precursor V with 2,3-dichloro-5,6-dicyano-1,4-benzoquinone (DDQ). In some embodiments, Compound I is formed by treating Precursor V with an α-bromination reagent, followed by elimination of an equivalent of HBr. In some embodiments, Compound I is formed by treating Precursor V with N-bromosuccinimide (NBS), followed by heating to form Compound I. In some embodiments, Compound I is formed by treating Precursor V with NBS in the presence of UV light to form Compound I. In some embodiments, Compound I is formed by treating Precursor V with NBS in the presence of UV light and with heating to form Compound I. In some embodiments, the UV light has a wavelength of 365 nm. In some embodiments, Compound I is formed by treating Precursor V with NBS in an organic solvent. In some embodiments, Compound I is formed by treating Precursor V with NBS in DCM. In some embodiments, Compound I is formed in the presence of a base. Non-limiting examples of bases include alkylamines, such as mono-, di, or trialkylamines (e.g., monoethylamine, diethylamine, triethylamine, and N,N-diisopropylethylamine (DIPEA)), pyridines, such as collidine and 4-dimethylaminopyridine (DMAP), and imidazoles, such as N-methylimidazole, as well as benzylamine, methylbenzylamine, morpholine, piperidine, picoline, dicyclohexylamine, N,N'-dibenzylethylenediamine, 2-hydroxyethylamine, bis-(2-hydroxyethy)amine, tri-(2-hydroxyethyl)amine, procaine, dibenzylpiperidine, dehydroabietylamine, *N,N'-*bisdehydroabietylamine, glucamine, N-methylglucamine, quinine, quinoline, lysine, arginine, 1,4-diazabicyclo[2.2.2]octane (DABCO), lutidine, poly(4-vinylpyridine), Na₂CO₃, K₂CO₃, CS₂CO₃, CaCO₃, LiH, NaH, KH, NaOH, LiOH, and KOH. In some cases, the base is a trialkylamine (e.g., triethylamine or N,N-diisopropylethylamine (DIPEA)), lutidine, collidine, Na₂CO₃, K₂CO₃, Cs₂CO₃, CaCO₃, LiH, NaH, or KH. In some embodiments, the formation of Compound I is carried out at elevated temperature. In some embodiments, the formation of Compound I is carried out at a temperature of 20 °C to 50 °C, for example, at least 20, 25, 30, 35, 40, 45, or 50 °C and/or up to 20, 25, 30, 35, 40, 45, or 50 °C, such as 20 °C to 50 °C, 30 °C to 50 °C, 40 °C to 50 °C, or 30 °C to 40 °C. In some embodiments, the formation of Compound I occurs at a temperature of 35 °C to 45 °C or 35°C to 40°C. In some embodiments, the formation of Compound I occurs at a temperature of about 40 °C.

The processes of the disclosure can include synthesizing Compound II:

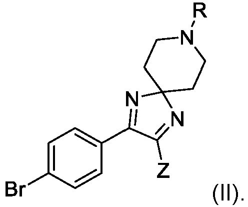

As used throughout, Z is a halogen or a sulfonate group. In some cases, Z is chloride. In some cases, Z is triflate.

In particular, Compound I can be admixed with a halogenation reagent or a sulfonylation reagent to form Compound II. Halogenation reagents and sulfonylation reagents are generally known in the art. In some cases, the halogenation reagent is a chlorination reagent. Nonlimiting examples of chlorination reagents that can be used throughout the processes described herein include SOCl₂, POCl₃, Vilsmeier reagents, oxalyl chloride, PCl₅, SOBr₂, and POBr₃. Nonlimiting examples of sulfonylation reagents that can be used throughout the processes described herein include triflic anhydride, mesyl chloride, mesic anhydride, tosyl chloride, tosic anhydride, benzenesulfonyl chloride, benzenesulfonic anhydride, nosyl chloride, and perfluoroalkylsulfonic anhydrides. In some embodiments, Compound I is admixed with a halogenation reagent to form Compound II. In some embodiments, Compound I is admixed with a chlorination reagent to form Compound II. In some embodiments, Compound I is admixed with a sulfonylation reagent to form Compound II. In some embodiments, Compound I is admixed with triflic anhydride to form Compound II.

In some embodiments, Compound II is formed by admixing Compound I with a halogenation or sulfonylation reagent in an organic solvent. In some embodiments, Compound II is formed by admixing Compound I with a halogenation or sulfonylation reagent in DCM. In some embodiments, Compound II is formed by admixing Compound I with a halogenation or sulfonylation reagent in ethyl acetate. In some embodiments, Compound II is formed by admixing Compound I with a halogenation or sulfonylation reagent in a mixture of organic solvents. In some embodiments, Compound II is formed by admixing Compound I with a halogenation or sulfonylation reagent in a mixture of DCM and ethyl acetate. In some embodiments, Compound II is formed by admixing Compound I with a chlorination or sulfonylation reagent in an organic solvent. In some embodiments, Compound II is formed by admixing Compound I with a chlorination or sulfonylation reagent in a mixture of organic solvents. In some embodiments, Compound II is formed by admixing Compound I with a chlorination or sulfonylation reagent in a mixture of DCM and ethyl acetate. In some embodiments, DCM and ethyl acetate are present in a volume ratio of 2:1, 1.9:1, 1.8:1, 1.7:1, 1.6:1, 1.5:1, 1.4:1, 1.3:1, 1.2:1, 1.1:1, or 1:1. In some embodiments, DCM and ethyl acetate are present in a volume ratio of 1.5:1.

In some embodiments, Compound II is formed by admixing Compound I with a chlorination or sulfonylation reagent in a mixture of DCM and ethyl acetate in the presence of a base. Non-limiting examples of bases include alkylamines, such as trialkylamines (e.g., triethylamine, or N,N-diisopropylethylamine (DIPEA)), pyridines, such as collidine and 4-diethylaminopyridine (DMAP), and imidazoles, such as N-methylimidazole, as well as dimethylbenzylamine, N-methylmorpholine, N-methylpiperidine, picoline, N-methyldicyclohexylamine, N,N'-dibenzylethylenediamine, tri-(2-hydroxyethyl)amine, procaine, dibenzylpiperidine, dehydroabietylamine, *N,N'*-bisdehydroabietylamine, N-methylglucamine, quinine, quinoline, lysine, arginine, 1,4-diazabicyclo[2.2.2]octane (DABCO), N,N-diisopropylethylamine, lutidine, poly(4-vinylpyridine), Na₂CO₃, K₂CO₃, CS₂CO₃, LiH, NaH, KH, NaOH, LiOH, and KOH. In some cases, the base is a trialkylamine (e.g., triethylamine or N,N-diisopropylethylamine (DIPEA)), lutidine, collidine, Na₂CO₃, K₂CO₃, CS₂CO₃, CaCO₃, LiH, NaH, or KH. In some cases, the base is a trialkyl amine, or more specifically, comprises triethylamine or DIPEA. In some embodiments, Compound II is formed by admixing Compound I with a chlorination or sulfonylation reagent in a mixture of DCM and ethyl acetate in the presence of DIPEA. In some embodiments, the base and Compound I are present in a molar ratio of 2:1, 1.9:1, 1.8:1, 1.7:1, 1.6:1, 1.5:1, 1.4:1, 1.3:1, 1.2:1, 1.1:1, or 1:1. In some embodiments, the base and Compound I are present in a molar ratio of 1.5:1. In some embodiments, DIPEA and Compound I are present in a molar ratio of 1.5:1.

In some embodiments, Compound II is formed by admixing Compound I with a chlorination or sulfonylation reagent in the presence of di-tert-butyl dicarbonate (Boc₂O), and optionally a base as described above. In some embodiments, Compound II is formed by admixing Compound I with a chlorination or sulfonylation reagent in a mixture of DCM and ethyl acetate in the presence of DIPEA and Boc₂O. In some embodiments, Boc₂O and Compound I are present in a molar ratio of 2:1, 1.9:1, 1.8:1, 1.7:1, 1.6:1, 1.5:1, 1.4:1, 1.3:1, 1.2:1, 1.1:1, or 1:1. In some embodiments, Boc₂O and Compound I are present in a molar ratio of 1:1.

In some embodiments, the formation of Compound II is carried out at elevated temperature. In some embodiments, the formation of Compound II is carried out at a temperature of 20 °C to 50 °C, for example, at least 20, 25, 30, 35, 40, 45, or 50 °C and/or up to 20, 25, 30, 35, 40, 45, or 50 °C, such as 20 °C to 50 °C, 30 °C to 50 °C, 40 °C to 50 °C, or 30 °C to 40 °C. In some embodiments, the formation of Compound II occurs at a temperature of 35 °C to 40 °C. In some embodiments, the formation of Compound II occurs at a temperature of 40 °C.

In some embodiments, Compound I and/or Compound II is present as a salt. A salt of Compound I, Compound II, or any other compound described herein (e.g., Compound A) can be prepared, for example, by reacting the compound in its free form with a suitable organic or inorganic acid, and optionally isolating the salt thus formed. Nonlimiting examples of suitable acid salts include hydrobromide, hydrochloride, sulfate, bisulfate, sulfonate, camphorsulfonate, phosphate, nitrate, acetate, valerate, oleate, palmitate, stearate, laurate, benzoate, lactate, phosphate, tosylate, citrate, maleate, fumarate, succinate, tartrate, naphthylate, mesylate, glucoheptonate, lactobionate, laurylsulfonate salts, and amino acid salts.

### Synthesis of Compound A via step (ii)(a)

The processes of the disclosure can include synthesizing Compound A via reaction of Compound II and Compound III (via step (ii)(a)) when R is methyl. The same process synthesizes Compound IV when R is a nitrogen-protecting group. In particular, the processes of the disclosure can include admixing Compound II and Compound III in the presence of a base to form Compound A either directly or indirectly via Compound IV:

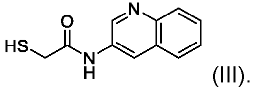

In some embodiments, the base is LiH, NaH, KH, LDA, KHMDS, NaHMDS, LiHMDS, Na₂CO₃, K₂CO₃, Cs₂CO₃, NaOH, LiOH, KOH, sodium methoxide, sodium ethoxide, sodium tert-butoxide or potassium tert-butoxide. In some embodiments, the base is NaH. In some embodiments, the base and Compound III are present in a molar ratio of 2:1, 1.9:1, 1.8:1, 1.7:1, 1.6:1, 1.5:1, 1.4:1, 1.3:1, 1.2:1, 1.1:1, or 1:1. In some embodiments, the base and Compound III are present in a molar ratio of 1:1.

In some embodiments, Compound II and Compound III are present in a molar ratio of 2:1, 1.9:1, 1.8:1, 1.7:1, 1.6:1, 1.5:1, 1.4:1, 1.3:1, 1.2:1, 1.1:1, or 1:1. In some embodiments, Compound II and Compound III are present in a molar ratio of 1:1.

In some embodiments, Compound A is formed by admixing Compound II, Compound III, and a base in an organic solvent. In some embodiments, the organic solvent is ethyl acetate, dichloromethane (DCM), acetonitrile, tetrahydrofuran (THF), tetrahydropyran, dimethylformamide (DMF), diethyl ether, dibutyl ether, diisopropyl ether, methyl tert-butyl ether (MTBE), 2-methyltetrahydrofuran (2-MeTHF), dimethyl sulfoxide (DMSO), 1,2-dimethoxyethane (1,2-DME), or 1,4-dioxane, or a combination thereof.

### Synthesis of Compound A via step (ii)(b)

The processes of the disclosure can include synthesizing Compound A via formation of Compound IIA then reaction with Compound IIIA (step (ii)(b)) when R is methyl. The same process synthesizes Compound IV when R is a nitrogen-protecting group. In particular, the processes of the disclosure can include admixing Compound II and a nucleophilic sulfuration reagent source to form Compound IIA then admixing Compound IIA with Compound IIIA in the presence of a base to form Compound A either directly or indirectly via Compound IV:

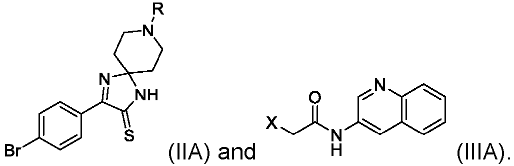

The processes of the disclosure can include synthesizing Compound IIA:

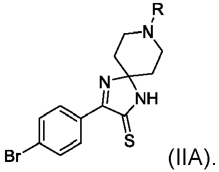

In particular, Compound II can be admixed with a nucleophilic sulfuration reagent to form Compound IIA.. Non-limiting examples of nucleophilic sulfuration reagents include Li₂S, Na₂S, K₂S, NaSH, AcSK, thiourea, Li₂Sₓ, Na₂Sₓ, K₂Sₓ, (wherein x = 2-5) and NaBH₄/S. In some embodiments, Compound IIA is formed by treating Compound II with Na₂S. In some embodiments, Compound IIA is formed by treating Compound II with a hydrate of Na₂S. In some embodiments, Compound IIA is formed by treating Compound II with Na₂S •4H₂O. In some embodiments, admixing Compound II and a nucleophilic sulfuration reagent occurs in the presence of a phase transfer catalyst. Non-limiting examples of phase transfer catalysts include tetrabutylammonium chloride (TBACI), tetrabutylammonium bromide (TBAB), and tetrabutylammonium iodide (TBAI). In some embodiments, the nucleophilic sulfuration reagent and Compound II are present in a molar ratio of 2:1, 1.9:1, 1.8:1, 1.7:1, 1.6:1, 1.5:1, 1.4:1, 1.3:1, 1.2:1, 1.1:1, or 1:1. In some embodiments, the nucleophilic sulfuration reagent and Compound II are present in a molar ratio of 1.5:1. In some embodiments, Compound IIA is formed by admixing the nucleophilic sulfuration reagent and Compound II in a mixture of organic solvents. In some embodiments, Compound IIA is formed by admixing the nucleophilic sulfuration reagent and Compound II in a mixture of DCM and ethyl acetate. In some embodiments, DCM and ethyl acetate are present in a volume ratio of 2:1, 1.9:1, 1.8:1, 1.7:1, 1.6:1, 1.5:1, 1.4:1, 1.3:1, 1.2:1, 1.1:1, or 1:1. In some embodiments, DCM and ethyl acetate are present in a volume ratio of 1.5:1.

The processes of the disclosure can include synthesizing Compound A via step (ii)(b). In particular, the processes of the disclosure can include admixing Compound IIA and Compound IIIA to form Compound A:

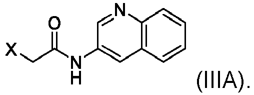

As used throughout, X is halogen. In some embodiments, X is I, Br, or Cl. In some embodiments, X is Br or Cl In some embodiments, X is Cl.

In particular, Compound IIA can be admixed with Compound IIIA to form Compound A.

In some embodiments, Compound A is formed by admixing Compound IIA and Compound IIIA in the presence of a base. In some embodiments, the base is NaH, Na₂CO₃, K₂CO₃, Cs₂CO₃, NaOH, LiOH, or KOH, or a combination thereof. In some embodiments, the base comprises K₂CO₃. In some embodiments, the base is a trialkylamine, pyridine, a pyridine derivative, or combinations thereof. In some embodiments, the base is a trialkylamine. In some embodiments, the trialkylamine is triethylamine or DIPEA. In some embodiments, the base and Compound IIA are present in a molar ratio of 5:1, 4:1, 3:1, 2:1, or 1:1. In some embodiments, the base and Compound IIA are present in a molar ratio of 2:1.

In some embodiments, Compound IIA, Compound IIIA, and a base are admixed in an organic solvent. In some embodiments, the organic solvent is ethyl acetate, dichloromethane (DCM), 2-butanone, acetonitrile, tetrahydrofuran (THF), tetrahydropyran, dimethylformamide (DMF), tetrahydrofurfuryl alcohol, diethyl ether, dibutyl ether, diisopropyl ether, methyl tert-butyl ether (MTBE), 2-methyltetrahydrofuran (2-MeTHF), dimethyl sulfoxide (DMSO), 1,2-dimethoxyethane (1,2-DME), 1,4-dioxane, methanol, ethanol, propanol, or 2-propanol, or a combination thereof. In some embodiments, the organic solvent comprises 2-propanol.

In some embodiments, admixing Compound IIA, Compound IIIA, and the base is carried out at elevated temperature. In some embodiments, the temperature is 20 °C to 50 °C, for example, at least 20, 25, 30, 35, 40, 45, or 50 °C and/or up to 20, 25, 30, 35, 40, 45, or 50 °C, such as 20 °C to 50 °C, 30 °C to 50 °C, 40 °C to 50 °C, or 30 °C to 40 °C. In some embodiments, the temperature is 40 °C to 45 °C. In some embodiments, the temperature is 45 °C.

### Synthesis of Compound A via step (iii)

The processes of the disclosure can include removing the nitrogen protecting group of R and methylating the intermediate amine to form Compound A where R is methyl (e.g., via step (iii) or form a compound intermediate where R is methyl (Compound II or Compound IIA). This modification of R from nitrogen protecting group to methyl group can occur for Compound II, Compound IIA, or Compound IV, e.g., can occur before or after any step of the disclosed processes. In some cases, the processes disclosed comprise converting Compound IV to Compound A.

Removal of the nitrogen-protecting group can comprise admixing the compound (e.g., Compound II, Compound IIA, or Compound IV) in the presence of acid. Nonlimiting examples of suitable acids include hydrobromic, hydrochloric, sulfuric, sulfonic, phosphoric, nitric, acetic, trifluoroacetic, benzoic, and tosic acid. In some embodiments, the acid comprises hydrochloric acid. In some embodiments, the acid and compound (Compound II, Compound IIA, or Compound IV) are present in a molar ratio of 20:1, 15:1, 12:1, 11:1, 10:1, 9:1, 8:1, 7:1, 6:1, 5:1, 4:1, 3:1, 2:1, or 1:1. In some embodiments, the acid and compound (Compound II, Compound IIA, or Compound IV) are present in a molar ratio of 10:1.

In some embodiments, removal of the nitrogen protecting group can occur in the presence of an acid and an organic solvent. In some embodiments, the organic solvent is dichloromethane (DCM), chloroform, 1,2-dichloroethane, ethyl acetate, acetonitrile, tetrahydrofuran (THF), tetrahydropyran, dimethylformamide (DMF), tetrahydrofurfuryl alcohol, diethyl ether, dibutyl ether, diisopropyl ether, methyl tert-butyl ether (MTBE), 2-methyltetrahydrofuran (2-MeTHF), dimethyl sulfoxide (DMSO), 1,2-dimethoxyethane (1,2-DME), 1,4-dioxane, methanol, ethanol, propanol, or 2-propanol, or a combination thereof. In some embodiments, the organic solvent comprises methanol.

Removal of the nitrogen-protecting group can comprise catalytic hydrogenation of the compound (e.g., Compound II, Compound IIA, or Compound IV). Non-limiting examples of suitable hydrogenation catalysts include nickel catalysts (e.g., Ni/ Al₂O₃), palladium catalysts (e.g., Pd/C) and platinum catalysts (e.g., Pt/C). In some embodiments, the hydrogenation catalyst is Pd/C.

In some embodiments, removal of the nitrogen protecting group can occur in the presence of hydrogen gas, a hydrogenation catalysts, and an organic solvent. In some embodiments, the organic solvent is dichloromethane (DCM), chloroform, 1,2-dichloroethane, tetrahydrofuran (THF), tetrahydropyran, tetrahydrofurfuryl alcohol, diethyl ether, dibutyl ether, diisopropyl ether, methyl tert-butyl ether (MTBE), 2-methyltetrahydrofuran (2-MeTHF), 1,2-dimethoxyethane (1,2-DME), 1,4-dioxane, methanol, ethanol, propanol, or 2-propanol, or a combination thereof. In some embodiments, the organic solvent comprises methanol.

In some embodiments, removal of the nitrogen protecting group is carried out at ambient temperature. In some embodiments, the temperature can be 20 °C to 30 °C, for example, at least 20, 25, or 30 °C and/or up to 20, 25, or 30°C, such as 20 °C, 21 °C, 22 °C, 23 °C, 24 °C, 25 °C, 26 °C, 27 °C, 28 °C, 29 °C, or 30 °C. In some embodiments, the temperature is 20 °C to 25 °C. In some embodiments, the temperature is 25 °C.

Methylation of the deprotected amine can be performed by admixing the deprotected amine with a methylating agent. In some embodiments, the processes comprise admixing the deprotected amine compound derived from Compound II, Compound IIA, or Compound IV with a methylating agent, and a base if required. Nonlimiting examples of suitable methylating agents include methyl iodide, dimethyl sulfate, methyl tosylate, methyl bromide, all in the presence of a suitable base, a combination of NaBH₃CN, CH₂O, and acetic acid, a combination of NaBH₄, CH₂O, and acetic acid, a combination of NaBH(OAc)₃, CH₂O, Et₃N, a combination of NaBH(OAc)₃, CH₂O, and acetic acid, or CH₂O and H₂ in presence of Ni, Pt, or Pd catalysts. In some embodiments, the methylating agent is a combination of NaBH(OAc)₃, CH₂O, and acetic acid. In some embodiments, the combination of NaBH(OAc)₃, CH₂O, and acetic acid is present in a molar ratio of 3:2:3 NaBH(OAc)₃:CH₂O:acetic acid per mole of deprotected amine derived from Compound II, Compound IIA, or Compound IV.

In some embodiments, the methylation is carried out at low temperature (e.g., a temperature below room temperature, 25°C). In some embodiments, the temperature is 0 °C to 20 °C, for example, at least 0, 5, 10, 15, or 20 °C and/or up to 0, 5, or 10°C, such as 0 °C, 1 °C, 2 °C, 3 °C, 4 °C, 5 °C, 6 °C, 7 °C, 8 °C, 9 °C, or 10 °C. In some embodiments, the temperature is 0 °C to 5 °C. In some embodiments, the temperature is 5 °C.

It is to be understood that while the disclosure is read in conjunction with the detailed description thereof, the foregoing description and following example are intended to illustrate and not limit the scope of the disclosure, which is defined by the scope of the appended claims. Other aspects, advantages, and modifications are within the scope of the appended claims.

### EXAMPLES

The following examples are provided for illustration and are not intended to limit the scope of the disclosure.

### Example 1: Synthesis of Compound II

**Precursor III** (2-amino-2-(4-bromophenyl)acetamide) was prepared according to the following reaction scheme:

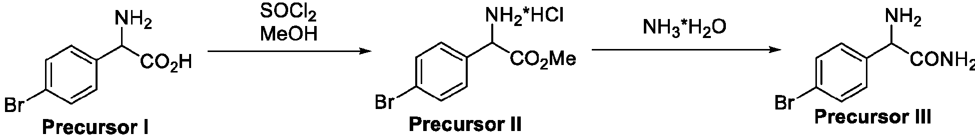

**Precursor I** (2-amino-2-(4-bromophenyl)acetic acid, 1.75 kg, 7.6 mol, 1.0 eq.) was charged into a 30 L reactor with methanol (12.3 L, 7 volumes) under nitrogen atmosphere. The nitrogen was purged three times, and the reaction mixture was cooled to 0 °C. To this solution was added SOCl₂ (1.36 kg, 11.4 mol, 1.5 eq.) dropwise to the solution at 10-20 °C. The reaction was stirred at 10-20 °C for 0.5 h, then warmed to 30-35 °C and stirred for 6 h. The solution was partially concentrated under reduced pressure to 5 L at 40 °C, MTBE was added (18 L, 10 v), and the mixture was stirred for 1 h at 5-10 °C. The solution was filtered, and the filter cake was washed with MTBE (3 L, 2 v). The filter cake was dried under reduced pressure at 40 °C to obtain **Precursor II** with 98 A% purity in an 82% yield.

**Precursor II** (1.75 kg, 6.2 mol, 1.0 eq.) was charged into a 20 L reactor with NH₃•H₂O (28%, 8.8 L, 5 v). The mixture was stirred at 10-15 °C for 20 h, then filtered and the filter cake washed with water (3.5 L, 2 v). The filter cake was dried under reduced pressure at 50 °C to obtain **Precursor III** (2-amino-2-(4-bromophenyl)acetamide) with 96 A% purity in an 85% isolated yield.

**Precursor V** (tert-butyl 2-(4-bromophenyl)-3-oxo-1,4,8-triazaspiro[4.5]decane-8-carboxylate) was prepared according to the following reaction scheme:

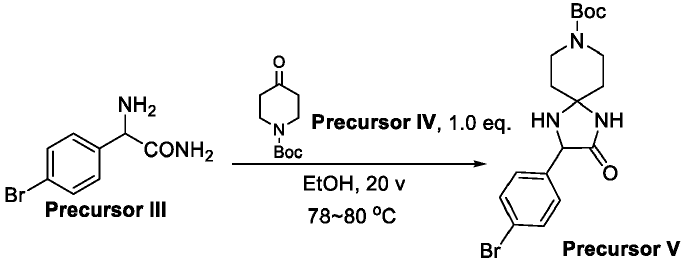

**Precursor III** (2-amino-2-(4-bromophenyl)acetamide, 500.0 g, 2.18 mol, 1.0 eq), **Precursor IV** (436.7 g, 2.18 mol, 1.0 eq.), and EtOH (10 L, 20 v) were charged into a 20 L reactor. The reaction reactor was purged with N₂ three times, and then the reaction mixture was heated to reflux and stirred for 16 h. The mixture was concentrated to ca. 2 v under reduced pressure at 45 °C, and then water (7 L, 7 v) was added and stirred at 15-20 °C for 0.5 h. The reaction mixture was filtered and the filter cake was washed with 2:7 EtOH/water (v/v, 1 L, 1 vol). The filter cake was dried under reduced pressure at 50 °C to give **Precursor V** (tert-butyl 2-(4-bromophenyl)-3-oxo-1,4,8-triazaspiro[4.5]decane-8-carboxylate) with 98% LC purity in a 76% isolated yield.

**Compound I** (tert-butyl 2-(4-bromophenyl)-3-oxo-1,4,8-triazaspiro[4.5]dec-1-ene-8-carboxylate) was synthesized according to the following reaction scheme:

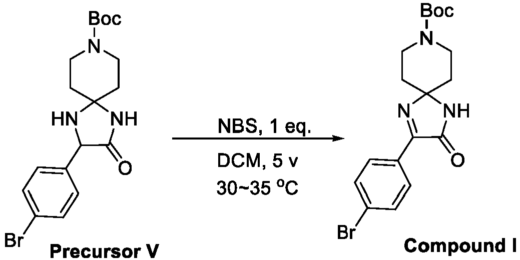

**Precursor V** ( tert-butyl 2-(4-bromophenyl)-3-oxo-1,4,8-triazaspiro[4.5]decane-8-carboxylate, 1.5 kg, 1.0 eq.), Na₂CO₃ (194 g, 0.5 eq.) and DCM (15 L, 10 v) were charged into a 30 L reactor, which was purged with N₂ three times and cooled to 0 °C. NBS (651.4 g, 1.0 eq.) was charged in four portions into the mixture. The reaction was stirred at 30-35 °C for 20 h. The reaction mixture was irradiated with UV light at 365 nm for 1 h, then stirred at 30-35 °C for 4 h. To this mixture was added 20 wt% aq. Na₂CO₃ (7.5 L, 5 v) and (Boc)₂O (80 g, 0.1 eq.) and the mixture was stirred for 30 min.

The reaction mixture was worked up as follows: The aqueous phase was separated and extracted with DCM (15 L x 2, 10 v x 2). The organic phase was concentrated to dryness under reduced pressure at 35-40 °C. The product was slurried in EtOH (4.5 L, 3 v), the mixture was filtered, and the filter cake was dried under reduced pressure at 35-40 °C to give **Compound I** (tert-butyl 2-(4-bromophenyl)-3-oxo-1,4,8-triazaspiro[4.5]dec-1-ene-8-carboxylate) with 96.4% HPLC purity in an 81% isolated yield.

**Compound II** (tert-butyl 2-(4-bromophenyl)-3-(((trifluoromethyl)sulfonyl)oxy)-1,4,8-triazaspiro[4.5]deca-1,3-diene-8-carboxylate) was synthesized according to the following reaction scheme:

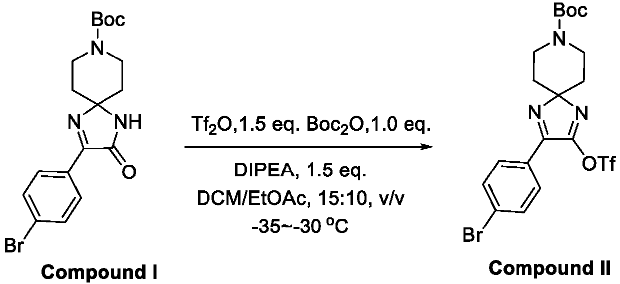

**Compound I** (tert-butyl 2-(4-bromophenyl)-3-oxo-1,4,8-triazaspiro[4.5]dec-1-ene-8-carboxylate, (600 g, 1.0 eq.), Boc₂O (320.8 g, 1.0 eq.), DCM (9.0 L, 15 v) and EtOAc (6.0 L, 10 v) were charged into a reactor under N₂. To this mixture was added DIPEA (284.3 g, 1.5 eq.). The mixture was purged with N₂ three times, the solution was cooled to -30 °C, and a solution of Tf₂O (620.7 g, 1.5 eq.) in DCM (1.2 L, 2 v) was added into the mixture at -30 °C. The mixture was stirred at -30 °C for 1 h to produce Compound II (tert-butyl 2-(4-bromophenyl)-3-(((trifluoromethyl)sulfonyl)oxy)-1,4,8-triazaspiro[4.5]deca-1,3-diene-8-carboxylate) in 90.2 A% purity, the solution of which was directly used in the next step.

### Example 2: Synthesis of Compound A via steps (ii)(b) and (iii)

**Compound IIA** (tert-butyl 2-(4-bromophenyl)-3-thioxo-1,4,8-triazaspiro[4.5]dec-1-ene-8-carboxylate) was synthesized according to the following reaction scheme:

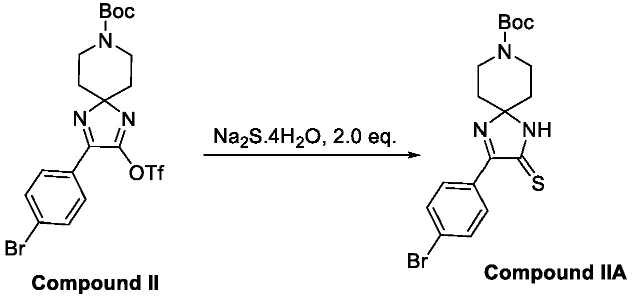

To a solution of **Compound II** (tert-butyl 2-(4-bromophenyl)-3-oxo-1,4,8-triazaspiro[4.5]dec-1-ene-8-carboxylate, in DCM (9.0 L, 15 v) and EtOAc (6.0 L, 10 v) under N₂ as prepared in Example 1 was added neat Na₂S•4H₂O (461.0 g, 2.0 eq.) and stirred at -30 °C for 1 h. To this solution was added water (10 L, 10 v) followed by EtOAc (10 L, 10 v) at -30 to -10 °C. This mixture was stirred for 0.5 h at -10 to 0 °C. The mixture was filtered and dried under reduced pressure at 40 °C giving **Compound IIA** (tert-butyl 2-(4-bromophenyl)-3-thioxo-1,4,8-triazaspiro[4.5]dec-1-ene-8-carboxylate) in 98% HPLC purity in an 82% corrected yield.

The methylated derivative of Compound IIA **("Compound IIA-Me",** 3-(4-bromophenyl)-8-methyl-1,4,8-triazaspiro[4.5]dec-3-ene-2-thione) was synthesized according to the following reaction scheme:

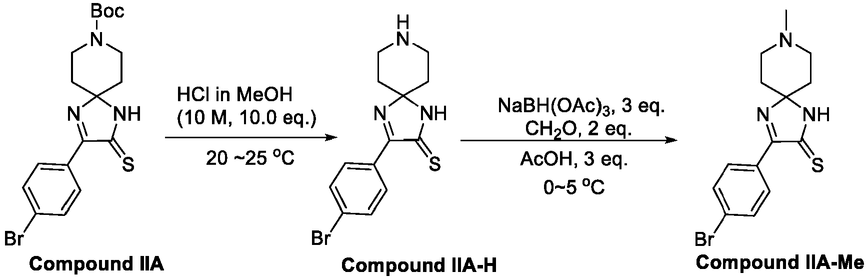

Under N₂, **Compound IIA** (tert-butyl 2-(4-bromophenyl)-3-thioxo-1,4,8-triazaspiro[4.5]dec-1-ene-8-carboxylate, 530 g, 1.0 eq.) and DCM (26.5 L, 50 v) were charged into a 30 L reactor. The solution was cooled to 0 °C, and HCl in MeOH (10 M, 1.25 L, 10.0 eq.) was added. The solution was warmed to 20-25 °C and stirred for 3 h. The mixture was filtered, and the filter cake was washed with DCM, then dried under reduced pressure at 40 °C for 6 h to afford **Compound IIA-H,** 3-(4-bromophenyl)-1,4,8-triazaspiro[4.5]dec-3-ene-2-thione as its hydrochloride salt with 98.1% HPLC purity in a 90% isolated yield.

Under N₂, **Compound IIA-H,** (3-(4-bromophenyl)-1,4,8-triazaspiro[4.5]dec-3-ene-2-thione hydrochloride 630 g, 1.0 eq.), CH₂O (283.5 g, 2.0 eq., 37 wt%), AcOH (315 g, 3.0 eq) and THF (9.5L, 15 v) were charged into a 20 L reactor. The solution was purged with N₂ three times, and then cooled to 0 °C. To this solution was added NaBH(OAc)₃ (1107 g, 3.0 eq) in 4 portions at 0 to 5 °C over 1 h. The solution was purged with N₂ three times and stirred at 0 °C for 1.5 h. To the solution was added sat. aq. Na₂CO₃ (15 L, 25 v) and then EtOAc (6.3 L, 10 v). This mixture was separated, and the organic phase was washed twice with sat. aq. Na₂CO₃ (6.3 L x 2, 10 v x 2), and then twice with brine (6.3 L x 2, 10 v x 2). The combined organic layers were combined and concentrated under reduced pressure at 40 °C to give **Compound IIA-Me** (3-(4-bromophenyl)-8-methyl-1,4,8-triazaspiro[4.5]dec-3-ene-2-thione) with 97.5% HPLC purity in an 86% isolated yield.

**Compound A** (2-((3-(4-bromophenyl)-8-methyl-1,4,8-triazaspiro[4.5]deca-1,3-dien-2-yl)thio)-N-(quinolin-3-yl)acetamide) was synthesized according to the following reaction scheme:

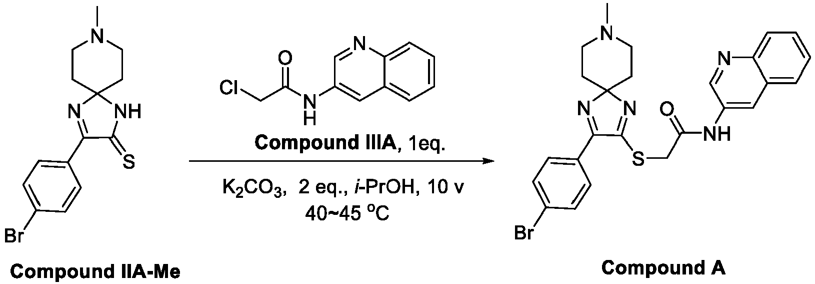

Under an atmosphere of N₂, **Compound IIA-Me** (3-(4-bromophenyl)-8-methyl-1,4,8-triazaspiro[4.5]dec-3-ene-2-thione, 500 g, 1.0 eq.), **Compound IIIA** (325.2 g, 1.0 eq.) and i-PrOH (12.3 L, 15 v) were charged into a 30 L reactor and purged with N₂ three times. To this solution was added aq. K₂CO₃ (2 M, 1.48 L, 2.0 eq.), and the solution was stirred at 40 °C for 4 h. A THF solution of Me₂NH (33 wt%, 1.00 kg) was added to the solution, and this mixture was stirred for 30 min. To this mixture was added water (2 L, 4 v), and the mixture was cooled to 10 °C and stirred for 1 h. The mixture was filtered and the filter cake washed with i-PrOH (500 mL, 1 v). The filter cake was dried under reduced pressure at 50 °C for 40 h to afford **Compound A** (2-((3-(4-bromophenyl)-8-methyl-1,4,8-triazaspiro[4.5]deca-1,3-dien-2-yl)thio)-N-(quinolin-3-yl)acetamide) with 99% LC purity in a 90% corrected (based on qNMR) yield.

**Compound IIIA** (2-chloro-N-(quinolin-3-yl)acetamide) was prepared as follows:
Quinolin-3-amine (285 g, 1.0 eq.), Na₂CO₃ (283 g, 1.0 eq.) and DCM (5.7 L, 20 v) were charged into a 10 L flask, which was purged with N₂ three times and cooled to -10 °C. To this solution was added 2-chloroacetyl chloride (356 g, 1.5 eq.) at -10 to 0 °C over 0.5 h, and the solution was stirred at 20-25 °C for 1.5 h. The mixture was filtered and the filter cake was washed with DCM (300 mL, 1 v). The filter cake was dissolved in EtOAc (1.5 L, 5 v) and the solution was washed with aq. Na₂CO₃ (600 mL, 2 v). The organic phase was separated and washed with brine (1.5 L, 5 v x 2), and then the organic phase was dried over Na₂SO₄ (600 g, 2 w). The solution was filtered, and the filter cake was washed with EtOAc (300 mL x 2, 1 v x 2). The filtrate was concentrated under reduced pressure at 45 °C to give Compound IIIA (2-chloro-N-(quinolin-3-yl)acetamide) with 98.3% LC purity in a 90% isolated yield.

### Example 3: Alternate Synthesis of Compound A via steps (ii)(b) and (iii)

**Compound I-Me** (3-(4-bromophenyl)-8-methyl-1,4,8-triazaspiro[4.5]dec-3-en-2-one) was synthesized according to the following reaction scheme:

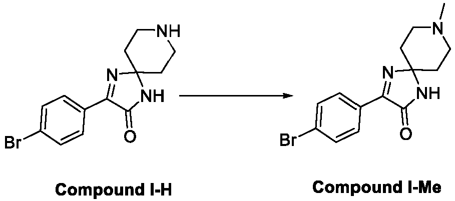

**Compound I-H** (3-(4-bromophenyl)-1,4,8-triazaspiro[4.5]dec-3-en-2-one) was synthesized as described in **Example 5,** below.

**Compound I-H** (3-(4-bromophenyl)-1,4,8-triazaspiro[4.5]dec-3-en-2-one, 1.0 g, 1.0 eq.), CH₂O (1.3 g, 5.0 eq., 37 w%), AcOH (584 mg, 3.0 eq.) and THF (10 mL) were charged into a flask under N₂, and the flask was purged with N₂ three times. To this mixture was added NaBH(OAc)₃ (2.1 g, 3.0 eq.), the flask was purged with N₂ three times, and the solution was stirred at 20 to 25 °C for 1.5 h. To this mixture was added saturated aqueous Na₂CO₃ (10 mL), followed by EtOAc (10 mL). The organic phase was separated and washed twice with saturated aqueous Na₂CO₃ (10 mL x 2), and then twice with brine (10 mL x 2). The organic layer was concentrated under reduced pressure at 40 °C to give 750 mg of **Compound I-Me** 3-(4-bromophenyl)-8-methyl-1,4,8-triazaspiro[4.5]dec-3-en-2-one with 99% HPLC purity in a 72% isolated yield.

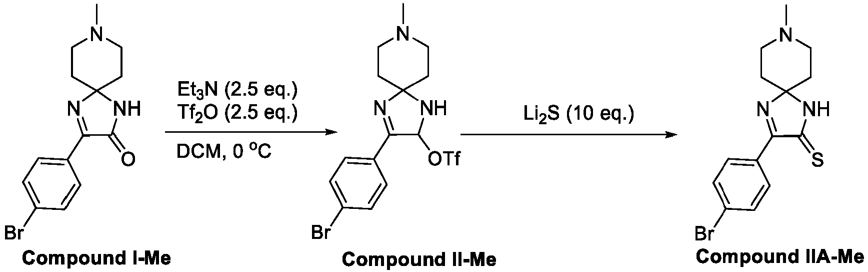

Under N₂, **Compound I-Me** (3-(4-bromophenyl)-8-methyl-1,4,8-triazaspiro[4.5]dec-3-en-2-one, 200 mg, 1.0 eq.) and DCM (2 mL) were charged into a flask, and to this mixture was added Et₃N (157 mg, 2.5 eq.). The flask was purged with nitrogen three times, and the solution was cooled to -10 °C. To this solution was added Tf₂O (326 mg, 2.5 eq) in DCM (0.4 mL) to the solution, and the solution was further cooled to -15 °C. The solution was stirred at -15 °C for 0.5 to afford **Compound II-Me,** 3-(4-bromophenyl)-8-methyl-1,4,8-triazaspiro[4.5]dec-3-en-2-yl trifluoromethanesulfonate.

To the solution of **Compound II-Me,** (3-(4-bromophenyl)-8-methyl-1,4,8-triazaspiro[4.5]dec-3-en-2-yl trifluoromethanesulfonate) was added Li₂S (286 mg, 10.0 eq), and the mixture was stirred at -10 °C for 1h. To the mixture was added water (2 mL) and then EtOAc (2 mL). The organic phase was separated and washed with water (2 mL) three times. The organic phase was concentrated to dryness under reduced pressure, and the product was slurried with EtOac (1 mL). The slurry was filtered and dried under reduced pressure at 40 °C to give **Compound IIA-Me** (3-(4-bromophenyl)-8-methyl-1,4,8-triazaspiro[4.5]dec-3-ene-2-thione) with 91% HPLC purity in a 36% corrected yield.

**Compound A** (2-((3-(4-bromophenyl)-8-methyl-1,4,8-triazaspiro[4.5]deca-1,3-dien-2-yl)thio)-N-(quinolin-3-yl)acetamide) was synthesized according to the following reaction scheme:

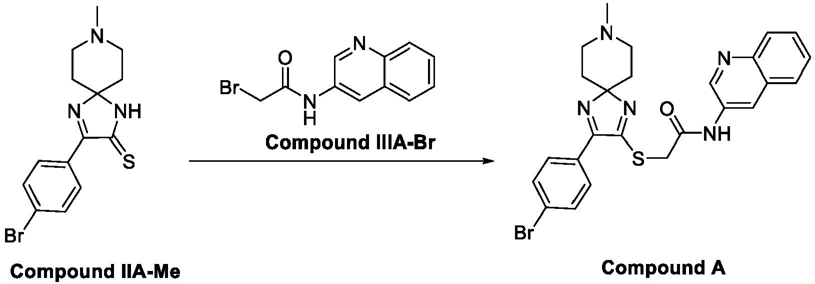

Under an atmosphere of N₂, **Compound IIA-Me** (3-(4-bromophenyl)-8-methyl-1,4,8-triazaspiro[4.5]dec-3-ene-2-thione, 100 mg, 1.0 eq.) and DCM (1 mL) were charged into a flask and purged with N₂ three times. To this solution was added **Compound IIIA-Br** (78 mg, 1.0 eq) and DIPEA (77 mg, 2.0 eq), and the solution was stirred at 20 °C for 5 h. To this mixture was added water (1 mL) and DCM (1 mL), and the organic phase was separated and washed with water (1 mL) three times. The mixture was concentrated to dryness under reduced pressure at 40 °C, and the mixture was purified by prep-TLC (eluting with 1:4 (v/v) DCM/MeOH) to afford 0.05 g of **Compound A** (2-((3-(4-bromophenyl)-8-methyl-1,4,8-triazaspiro[4.5]deca-1,3-dien-2-yl)thio)-N-(quinolin-3-yl)acetamide) with 99% HPLC purity in a 32% corrected yield.

### Example 4: Synthesis of Compound A-Boc via steps (ii)(b) and (iii)

**Compound A-Boc** (tert-butyl 2-(4-bromophenyl)-3-((2-oxo-2-(quinolin-3-ylamino)ethyl)thio)-1,4,8-triazaspiro[4.5]deca-1,3-diene-8-carboxylate) was synthesized according to the following reaction scheme:

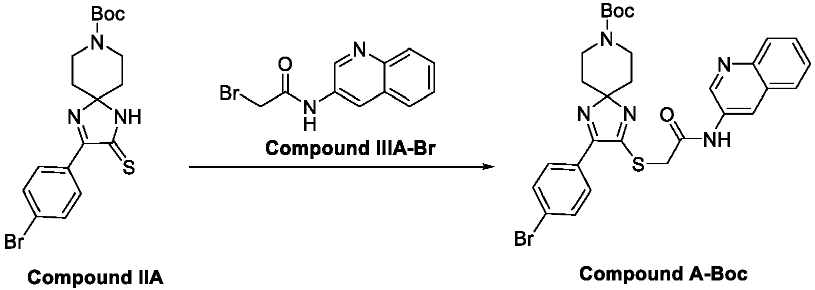

Under an atmosphere of N₂, **Compound IIA**(tert-butyl 2-(4-bromophenyl)-3-thioxo-1,4,8-triazaspiro[4.5]dec-1-ene-8-carboxylate, 200 mg, 1.0 eq.) and DCM (2 mL) were charged into a flask and purged with N₂ three times. To this solution was added **Compound IIIA-Br** (150 mg, 1.2 eq) and Et₃N (95 mg, 2.0 eq), and the solution was stirred at 20 °C for 1.5 h. To this mixture was added water (2 mL) and DCM (2 mL), and the organic phase was separated and washed with water (2 mL) three times. The mixture was concentrated to dryness under reduced pressure at 40 °C to afford 250 mg of **Compound A-Boc** (tert-butyl 2-(4-bromophenyl)-3-((2-oxo-2-(quinolin-3-ylamino)ethyl)thio)-1,4,8-triazaspiro[4.5]deca-1,3-diene-8-carboxylate) with 85 A% LC purity in a 87% corrected yield.

### Example 5: Synthesis of Compound A-Bn via steps (ii)(b) and (iii)

Compound I-H (3-(4-bromophenyl)-1,4,8-triazaspiro[4.5]dec-3-en-2-one) was synthesized according to the following reaction scheme:

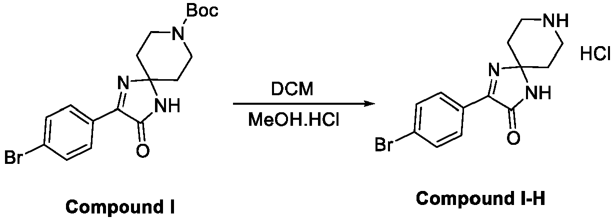

**Compound I** (tert-butyl 2-(4-bromophenyl)-3-oxo-1,4,8-triazaspiro[4.5]dec-1-ene-8-carboxylate, 55 g, 1.0 eq.) and DCM (2.7 L) were charged into a flask under N₂, and the solution was cooled to 0 °C. To this solution was added HCl in MeOH (10 M, 115 mL, 10.0 eq.), and the mixture was warmed to 20 to 25 °C and stirred for 12 h. The mixture was filtered, and the filter cake was washed with DCM (110 mL). The filter cake was dried under reduced pressure at 40 °C for 6 h to afford 45 g of **Compound I-H** 3-(4-bromophenyl)-1,4,8-triazaspiro[4.5]dec-3-en-2-one with 94% LC purity in a 97% isolated yield.

**Compound I-Bn** (8-benzyl-3-(4-bromophenyl)-1,4,8-triazaspiro[4.5]dec-3-en-2-one) was synthesized according to the following reaction scheme:

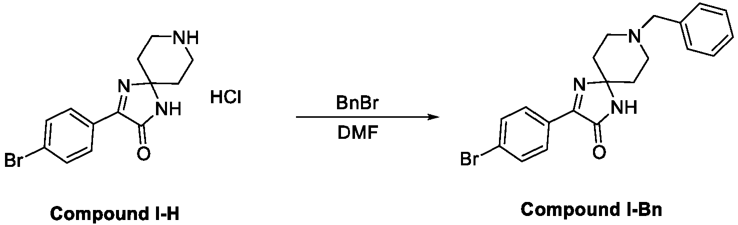

**Compound I-H** (3-(4-bromophenyl)-1,4,8-triazaspiro[4.5]dec-3-en-2-one, 45 g, 1.0 eq.), Na₂CO₃ (16.6 g, 1.2 eq.) and DMF (1.1 L, 25 v) were charged into a flask under N₂, and the solution was cooled to -5 °C. To this solution was added benzyl bromide (26.8 g, 1.2 eq.), and the mixture was warmed to 20 to 25 °C and stirred for 2 h. To this mixture was added water (450 mL) and then EtOAc (450 mL). The organic phase was separated and washed with water (450 mL) two times, then concentrated to dryness under reduced pressure at 40 °C for 6 h to afford 48 g of **Compound I-Bn** 8-benzyl-3-(4-bromophenyl)-1,4,8-triazaspiro[4.5]dec-3-en-2-one with 94% LC purity in an 82% isolated yield.

**Compound IIA-Bn** (8-benzyl-3-(4-bromophenyl)-1,4,8-triazaspiro[4.5]dec-3-ene-2-thione) was synthesized according to the following reaction scheme:

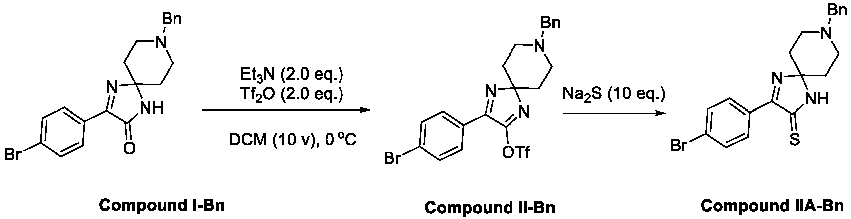

**Compound I-Bn** (8-benzyl-3-(4-bromophenyl)-1,4,8-triazaspiro[4.5]dec-3-en-2-one, 500 mg, 1.0 eq.) and DCM (5 mL) were charged into a flask under N₂. To this solution was added Et₃N (254 mg, 2.0 eq.), and the flask was purged with N₂ three times. The solution was cooled to 0 °C, to this mixture was added a solution of Tf₂O (528 mg, 2.0 eq.) in DCM (1 mL). This mixture was stirred at 0 °C for 1h to yield **Compound II-Bn** (8-benzyl-3-(4-bromophenyl)-1,4,8-triazaspiro[4.5]deca-1,3-dien-2-yl trifluoromethanesulfonate) which was used directly in the next step without purification.

To the solution of **Compound II-Bn** from the previous step was added Na₂S (1.96 g, 10.0 eq.), and the mixture was stirred at 0 °C for 1 h. To this solution was added water (5 mL) and then EtOAc (5 mL). The organic phase was separated and washed with water (5 mL) three times, then concentrated to dryness under reduced pressure at 40 °C. This residue was purified by silica gel column chromatography (eluting with 2:1 (v/v) n-heptane/EtOAc) to give 210 mg of **Compound IIA-Bn** 8-benzyl-3-(4-bromophenyl)-1,4,8-triazaspiro[4.5]dec-3-ene-2-thione with 97 A% purity in a 40% yield from **Compound I-Bn.**

**Compound A-Bn** (2-((8-benzyl-3-(4-bromophenyl)-1,4,8-triazaspiro[4.5]deca-1,3-dien-2-yl)thio)-N-(quinolin-3-yl)acetamide) was synthesized according to the following reaction scheme:

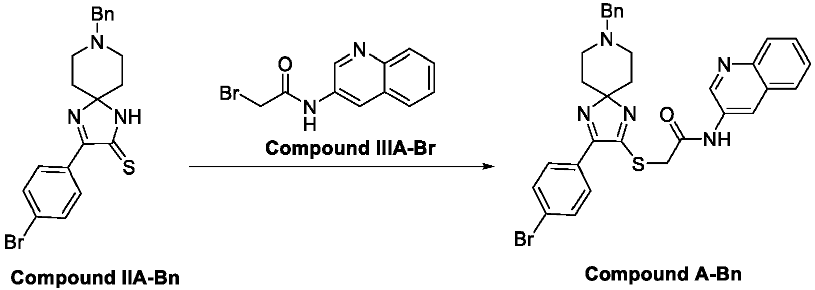

Under an atmosphere of N₂, **Compound IIA-Bn** (8-benzyl-3-(4-bromophenyl)-1,4,8-triazaspiro[4.5]dec-3-ene-2-thione, 150 mg, 1.0 eq.) and DCM (1.5 mL) were charged into a flask and purged with N₂ three times. To this solution was added **Compound IIIA-Br** (101 mg, 1.05 eq) and Et₃N (73 mg, 2.0 eq), and the solution was stirred at 20 °C for 5 h. To this mixture was added water (1.5 mL) and DCM (1.5 mL), and the organic phase was separated and washed with water (1.5 mL) three times. The mixture was concentrated to dryness under reduced pressure at 40 °C and purified by column chromatography (elute with 0-20% MeOH in DCM) to afford 0.1 g of **Compound A-Bn** (2-((8-benzyl-3-(4-bromophenyl)-1,4,8-triazaspiro[4.5]deca-1,3-dien-2-yl)thio)-N-(quinolin-3-yl)acetamide) with 99% HPLC purity in a 46% corrected yield.

## Claims

1. A process of synthesizing Compound A, or a salt thereof: 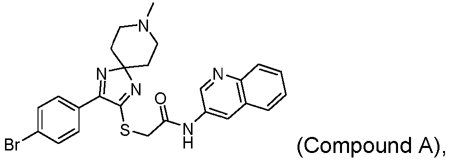 comprising
(i) admixing Compound I and a halogenation reagent or a sulfonylation reagent to form Compound II: 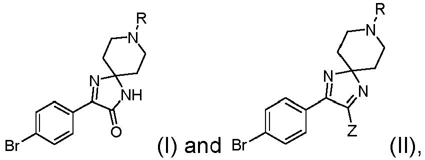 wherein R is methyl or a nitrogen-protecting group and Z is a halogen or a sulfonate group;
(ii)
(a) admixing Compound II and Compound III in the presence of a base to form Compound A: 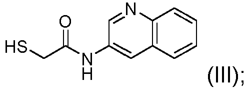 or
(b)(I) admixing Compound II and a nucleophilic sulfuration reagent to form Compound IIA, then (II) admixing Compound IIA with Compound IIIA in the presence of a base to form Compound A: 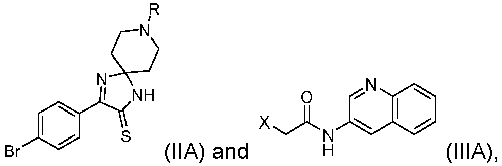 wherein X is halogen, and
(iii) optionally, when R is a nitrogen-protecting group, removing the nitrogen-protecting group and methylating the resulting deprotected amine to form Compound A wherein R is methyl.

2. The process of claim 1, wherein R is methyl.

3. The process of claim 1, wherein R is a nitrogen-protecting group, optionally t-butyloxycarbonyl (Boc), benzyloxycarbonyl(Cbz), benzyl, or p-methoxybenzyl (PMB), preferably t-butyloxycarbonyl (Boc).

4. The process of any one of claims 1 to 3, wherein Z is halogen, optionally chloride.

5. The process of any one of claims 1 to 4, wherein the halogenation reagent is a chlorination reagent, optionally wherein the chlorination reagent comprises oxalyl chloride, SOCl₂ or POCl₃.

6. The process of any one of claims 1 to 3, wherein Z is a sulfonate group, optionally triflate, mesylate, tosylate, benzenesulfonate, or nosylate, preferably triflate.

7. The process of claim 6, wherein the sulfonate group is formed by treating Compound I with a sulfonylation reagent selected from triflic anhydride, mesyl chloride, mesic anhydride, tosyl chloride, tosic anhydride, nosyl chloride, and a perfluoroalkylsulfonic anhydride, optionally wherein the sulfonate group is formed by treating Compound I with a triflic anhydride in a mixture of dichloromethane and ethyl acetate.

8. The process of any one of claims 1 to 7, wherein X is Br or Cl, optionally Cl.

9. The process of any one of claims 1 to 8, comprising step (ii)(a), optionally wherein the base is NaH, NaOH, KOH, sodium methoxide, sodium ethoxide, sodium tert-butoxide, or potassium tert-butoxide.

10. The process of any one of claims 1 to 8, comprising step (ii)(b), optionally wherein the nucleophilic sulfuration reagent comprises Li₂S, Na₂S, K₂S, Li₂Sₓ, Na₂Sₓ, K₂Sₓ, (wherein x = 2-5), NaBH₄/S, NaSH, AcSK, thiourea or a salt or hydrate thereof, preferably wherein the nucleophilic sulfuration reagent comprises a hydrate of Na₂S, and more preferably wherein the nucleophilic sulfuration reagent comprises Na₂S•4H₂O.

11. The process of claim 10, comprising admixing Compound II with Na₂S 4H₂O in a mixture of dichloromethane and ethyl acetate.

12. The process of claim 10 or 11, comprising admixing Compound IIA with Compound IIIA in the presence of K₂CO₃ to form Compound A, optionally wherein the step of admixing Compound IIA with Compound IIIA in the presence of K₂CO₃ to form Compound A is performed in isopropanol.

13. The process of any one of claims 1 and 3 to 12, wherein R is a nitrogen-protecting group and the process comprises step (iii), optionally wherein step (iii) is performed between steps (ii)(b)(I) and (ii)(b)(II).

14. The process of any one of claims 1 and 3 to 13, wherein R is a nitrogen-protecting group and the process further comprises removing the nitrogen-protecting group from Compound IIA and methylating the resulting deprotected amine to form Compound IIA wherein R is methyl.

15. The process of claim 13 or 14, wherein removing the nitrogen-protecting group comprises admixing in the presence of acid, and/or wherein the deprotected amine is methylated by admixing with NaBH(OAc)₃, CH₂O, and acetic acid.

## Patentansprüche

1. Prozess zum Synthetisieren von Verbindung A oder eines Salzes davon: 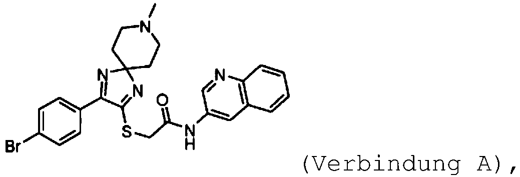 umfassend
(i) Vermischen von Verbindung I und eines Halogenierungsreagens oder eines Sulfonylierungsreagens, um Verbindung II zu bilden: 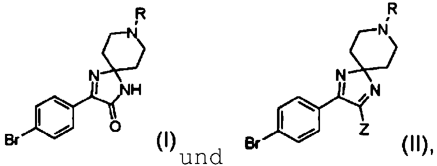 wobei R Methyl oder eine Stickstoffschutzgruppe ist und Z ein Halogen oder eine Sulfonatgruppe ist;
(ii)
(a) Vermischen von Verbindung II und Verbindung III in Gegenwart einer Base, um Verbindung A zu bilden: 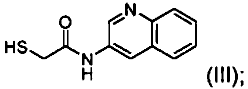 oder
(b) (I) Vermischen von Verbindung II und eines nukleophilen Schwefelungsreagens, um Verbindung IIA zu bilden, dann (II) Vermischen von Verbindung IIA mit Verbindung IIIA in Gegenwart einer Base, um Verbindung A zu bilden: 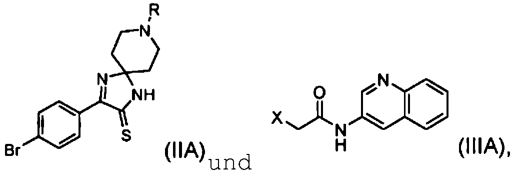 wobei X Halogen ist, und
(iii) gegebenenfalls, wenn R eine Stickstoffschutzgruppe ist, Entfernen der Stickstoffschutzgruppe und Methylieren des resultierenden entschützten Amins, um Verbindung A zu bilden, wobei R Methyl ist.

2. Prozess nach Anspruch 1, wobei R Methyl ist.

3. Prozess nach Anspruch 1, wobei R eine Stickstoffschutzgruppe ist, gegebenenfalls t-Butylcarbonyl (Boc), Benzyloxycarbonyl (Cbz), Benzyl oder p-Methoxybenzyl (PMB), vorzugsweise t-Butyloxycarbonyl (Boc).

4. Prozess nach einem der Ansprüche 1 bis 3, wobei Z Halogen ist, gegebenenfalls Chlorid.

5. Prozess nach einem der Ansprüche 1 bis 4, wobei das Halogenierungsreagens ein Chlorierungsreagens ist, wobei gegebenenfalls das Chlorierungsreagens Oxalylchlorid, SOCl₂ oder POCl₃ umfasst.

6. Prozess nach einem der Ansprüche 1 bis 3, wobei Z eine Sulfonatgruppe ist, gegebenenfalls Triflat, Mesylat, Tosylat, Benzolsulfonat oder Nosylat, vorzugsweise Triflat.

7. Prozess nach Anspruch 6, wobei die Sulfonatgruppe durch Behandeln von Verbindung I mit einem Sulfonylierungsreagens gebildet wird, das ausgewählt ist aus Trifluormethansulfonsäureanhydrid, Methansulfonsäurechlorid, Methansulfonsäureanhydrid, p-Toluolsulfonsäurechlorid, p-Toluolsulfonsäureanhydrid, 4-Nitrobenzolsulfonylchlorid und einem Perfluoralkylsulfonsäureanhydrid, wobei gegebenenfalls die Sulfonatgruppe durch Behandeln von Verbindung I mit einem Trifluormethansulfonsäureanhydrid in einem Gemisch aus Dichlormethan und Ethylacetat gebildet wird.

8. Prozess nach einem der Ansprüche 1 bis 7, wobei X Br oder Cl ist, gegebenenfalls Cl.

9. Prozess nach einem der Ansprüche 1 bis 8, umfassend Schritt (ii) (a), wobei gegebenenfalls die Base NaH, NaOH, KOH, Natriummethoxid, Natriumethoxid, Natrium-tert-butoxid oder Kalium-tert-butoxid ist.

10. Prozess nach einem der Ansprüche 1 bis 8, umfassend Schritt (ii) (b), wobei gegebenenfalls das nukleophile Schwefelungsreagens Li₂S, Na₂S, K₂S, Li₂Sₓ, Na₂Sₓ, K₂Sₓ (wobei x = 2-5), NaBH₄/S, NaSH, AcSK, Thioharnstoff oder ein Salz oder Hydrat davon umfasst, wobei vorzugsweise das nukleophile Schwefelungsreagens ein Hydrat von Na₂S umfasst und wobei weiter bevorzugt das nukleophile Schwefelungsreagens Na₂S ·4HS₂O umfasst.

11. Prozess nach Anspruch 10, umfassend Vermischen von Verbindung II mit Na₂S·4H₂O in einem Gemisch aus Dichlormethan und Ethylacetat.

12. Prozess nach Anspruch 10 oder 11, umfassend Vermischen von Verbindung IIA mit Verbindung IIIA in Gegenwart von K₂CO₃, um Verbindung A zu bilden, wobei gegebenenfalls der Schritt des Vermischens von Verbindung IIA mit Verbindung IIIA in Gegenwart von K₂CO₃, um Verbindung A zu bilden, in Isopropanol durchgeführt wird.

13. Prozess nach einem der Ansprüche 1 und 3 bis 12, wobei R eine Stickstoffschutzgruppe ist und der Prozess Schritt (iii) umfasst, wobei gegebenenfalls Schritt (iii) zwischen den Schritten (ii) (b) (I) und (ii) (b) (II) durchgeführt wird.

14. Prozess nach einem der Ansprüche 1 und 3 bis 13, wobei R eine Stickstoffschutzgruppe ist und der Prozess ferner Entfernen der Stickstoffschutzgruppe aus Verbindung IIA und Methylieren des resultierenden entschützten Amins umfasst, um Verbindung IIA zu bilden, wobei R Methyl ist.

15. Prozess nach Anspruch 13 oder 14, wobei das Entfernen der Stickstoffschutzgruppe Vermischen in der Gegenwart einer Säure umfasst und/oder wobei das entschützte Amin durch Vermischen mit NaBH(OAc)₃, CH₂O und Essigsäure methyliert wird.

## Revendications

1. Procédé de synthèse du composé A, ou d'un sel de celui-ci : 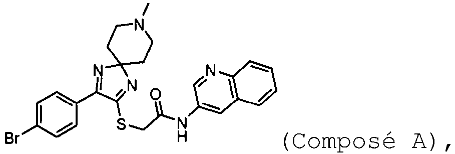 comprenant
(i) le mélange du composé I et d'un réactif d'halogénation ou d'un réactif de sulfonylation pour former le composé II : 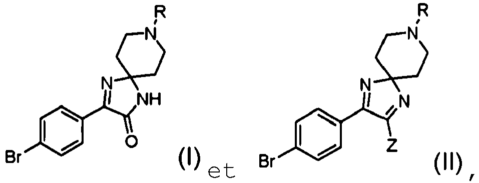 dans lequel R est un groupe méthyle ou un groupe protecteur de l'azote et Z est un halogène ou un groupe sulfonate ;
(ii)
(a) le mélange du composé II et du composé III en présence d'une base pour former le composé A : 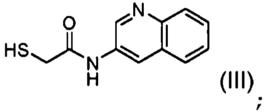 ou
(b) (I) le mélange du composé II et d'un réactif de sulfuration nucléophile pour former le composé IIA, puis (II) le mélange du composé IIA et du composé IIIA en présence d'une base pour former le composé A : 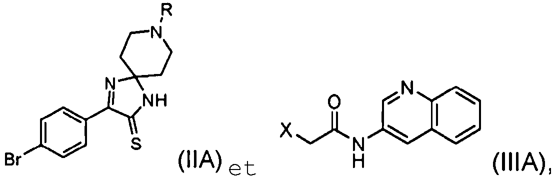 dans lequel X représente halogène, et
(iii) éventuellement, lorsque R est un groupe protecteur d'azote, la suppression du groupe protecteur d'azote et la méthylation de l'amine déprotégée résultante pour former le composé A dans lequel R est méthyle.

2. Procédé selon la revendication 1, dans lequel R est méthyle.

3. Procédé selon la revendication 1, dans lequel R est un groupe protecteur d'azote, éventuellement t-butyloxycarbonyle (Boc), benzyloxycarbonyle (Cbz), benzyle, ou p-méthoxybenzyle (PMB), de préférence t-butyloxycarbonyle (Boc).

4. Procédé selon l'une quelconque des revendications 1 à 3, dans lequel Z est halogène, éventuellement chlorure.

5. Procédé selon l'une quelconque des revendications 1 à 4, dans lequel le réactif d'halogénation est un réactif de chloration, éventuellement dans lequel le réactif de chloration comprend le chlorure d'oxalyle, SOCl₂ ou POCl₃.

6. Procédé selon l'une quelconque des revendications 1 à 3, dans lequel Z est un groupe sulfonate, éventuellement triflate, mésylate, tosylate, benzènesulfonate ou nosylate, de préférence triflate.

7. Procédé selon la revendication 6, dans lequel le groupe sulfonate est formé en traitant le composé I avec un réactif de sulfonation choisi parmi l'anhydride triflique, le chlorure de mésyle, l'anhydride mésique, le chlorure de tosyle, l'anhydride tosique, le chlorure de nosyle et un anhydride perfluoroalkylsulfonique, eventuellement dans lequel le groupe sulfonate est formé en traitant le composé I avec un anhydride triflique dans un mélange de dichlorométhane et d'acétate d'éthyle.

8. Procédé selon l'une quelconque des revendications 1 à 7, dans lequel X est Br ou Cl, éventuellement Cl.

9. Procédé selon l'une quelconque des revendications 1 à 8, comprenant l'étape (ii) (a), éventuellement dans lequel la base est NaH, NaOH, KOH, le méthylate de sodium, l'éthylate de sodium, le tert-butoxyde de sodium ou le tert-butoxyde de potassium.

10. Procédé selon l'une quelconque des revendications 1 à 8, comprenant l'étape (ii) (b), dans lequel le réactif de sulfuration nucléophile comprend éventuellement Li₂S, Na₂S, K₂S, Li₂Sₓ, Na₂Sₓ, K₂Sₓ, (où x = 2 à 5), NaBH₄/S, NaSH, AcSK, la thiourée ou un sel ou un hydrate de celui-ci, de préférence dans lequel le réactif de sulfuration nucléophile comprend un hydrate de Na₂S, et plus particulièrement dans lequel le réactif de sulfuration nucléophile comprend Na₂S·4H₂O.

11. Procédé selon la revendication 10, comprenant le mélange du composé II avec Na₂S 4H₂O dans un mélange de dichlorométhane et d'acétate d'éthyle.

12. Procédé selon la revendication 10 ou 11, comprenant le mélange du composé IIA avec le composé IIIA en présence de K₂CO₃ pour former le composé A, dans lequel l'étape de mélange du composé IIA avec le composé IIIA en présence de K₂CO₃ pour former le composé A est éventuellement réalisée dans l'isopropanol.

13. Procédé selon l'une quelconque des revendications 1 et 3 à 12, dans lequel R est un groupe protecteur d'azote et le procédé comprend l'étape (iii), éventuellement dans lequel l'étape (iii) est effectuée entre les étapes (ii) (b) (I) et (ii) (b) (II) .

14. Procédé selon l'une quelconque des revendications 1 et 3 à 13, dans lequel R est un groupe protecteur d'azote et le procédé comprend en outre l'élimination du groupe protecteur d'azote du composé IIA et la méthylation de l'amine déprotégée résultante pour former le composé IIA dans lequel R est un méthyle.

15. Procédé selon la revendication 13 ou 14, dans lequel l'élimination du groupe protecteur d'azote comprend un mélange en présence d'acide, et/ou dans lequel l'amine déprotégée est méthylée par mélange avec NaBH(OAc)₃, CH₂O et l'acide acétique.
